# EUROPEAN PATENT APPLICATION

(11) **EP 2 787 090 A1**
(43) Date of publication of application: **08.10.2014**
(21) Application number: 12854153.9
(22) Date of filing: 30.11.2012
(51) Int. Cl.: C21D 8/10, B21C 1/00, C22C 38/00, C22C 38/22

(54) **METHOD FOR ROLLING/DRAWING NICKEL-FREE HIGH-NITROGEN STAINLESS STEEL MATERIAL, NICKEL-FREE HIGH-NITROGEN STAINLESS STEEL SEAMLESS THIN PIPE, AND METHOD FOR PRODUCING SAME**

(30) Priority: 30.11.2011 JP 2011261664
(71) Applicant: National Institute for Materials Science, Tsukuba-shi, Ibaraki 305-0047 (JP)
(72) Inventor: TAGUCHI Tetsushi, Tsukuba-shi Ibaraki 305-0047 (JP); KATADA Yasuyuki, Tsukuba-shi Ibaraki 305-0047 (JP)
(74) Representative: Calamita, Roberto
(86) International application number: PCT/JP2012/081186
(87) International publication number: WO 2013/081144

(57) **Abstract**

Provided is a method of rolling/drawing a nickel-free high-nitrogen stainless steel material, the method including: an intermediate annealing process S11 in which a nickel-free high-nitrogen stainless steel material made of a fine grain structure having a maximum crystal grain size of 30 µm or less is annealed at a temperature of 900°C or higher and 1000°C or lower and then is air-cooled to room temperature; a rolling/drawing process S12 in which the nickel-free high-nitrogen stainless steel material is extended while being thinned or being reduced in diameter; and a final solution treatment process S13 in which the nickel-free high-nitrogen stainless steel material is heated to a temperature of 1200°C or higher and 1400°C or lower and then is air-cooled to room temperature. A nickel-free high-nitrogen stainless steel raw tube made of a fine grain structure 11 having a maximum crystal grain size of 30 µm or less is subjected to plastic working according to the method of rolling/drawing the nickel-free high-nitrogen stainless steel material to obtain a thin seamless tube 10 for the nickel-free high-nitrogen stainless steel stent. The thin seamless tube for the nickel-free high-nitrogen stainless steel stent is made of a fine grain structure having a maximum crystal grain size of 30 µm or less.

## Description

### Technical Field

The present invention relates to a method of rolling/drawing a nickel-free high-nitrogen stainless steel material, a thin seamless tube of nickel-free high-nitrogen stainless steel, and a method of manufacturing the same.

### Background Art

A coronary-artery stent is a metallic tubular article and is an instrument which is placed in a narrowed part or an occluded part of a coronary artery and is used to support the coronary artery such that the coronary artery is pressed and expanded from the inside thereof. The coronary-artery stent has features to reduce an acute coronary occlusion, to broaden an intravascular lumen, and to reduce chronic restenosis.

Biomaterials used in the coronary-artery stent require excellent resistance to corrosion in human body environment, safety for Ni (nickel) allergy or the like. Among lots of high-nitrogen stainless steel with excellent resistance to corrosion in human body environment, a high-nitrogen stainless steel not containing nickel (hereinafter, referred to as nickel-free) does not have a risk of causing Ni allergy or inflammation reaction, which is a promising material as the biomaterial (Patent Literature 1 and Non-Patent Literatures 1 and 2).

However, although the nickel-free high-nitrogen stainless steel has a fine grain structure, crystal grains are coarsened (grain growth) by repetition heat-treatment after a phase transformation in the case of working with a large working strain (Patent Literature 2 and Non-Patent Literature 3). For example, in the case of producing a thin seamless tube which can be worked into the coronary-artery stent, crystal grains are coarsened (grain growth), and thus the working of the coronary-artery stent having a complicated shape is difficult. For this reason, the stent may be damaged due to a stress concentration on crystal grain boundaries in the case of applying to the human body.

### Citation List

### Patent Literature

1: JP 4845109 B1
2: JP 2006-316338 A

### Non- Patent Literature

1: J. Menzel, W. Kirschner and G. Stein, High-nitrogen containing Ni-free austenitic steels for medical applications, ISIJ International, 36-7 (1996), pp. 893-900
2: G. Stein, I. Hucklenbroich and H. Feichtinger, Current and future applications of high-nitrogen steels, Material Science Forum, 318-320 (1999), pp. 151-160
3: T. ONOMOTO, Y. TERAZAWA, T. TSUCHIYAMA and S. TAKAKI, Effect of Grain Refinement on Tensile Properties in Fe-25Cr-1 N Alloy, ISIJ International, 49-8 (2009), pp. 1246-1252

### Summary of Invention

### Technical Problem

An object of the present invention is to provide a method of rolling/drawing a nickel-free high-nitrogen stainless steel material, which does not increase a grain size of a fine grain structure even in the case of working with a larger working strain, a thin seamless tube of nickel-free high-nitrogen stainless steel having a small grain size of the fine grain structure, and a method of manufacturing the same.

### Solution to Problem

The present inventors found an annealing temperature (heat-treatment temperature) and rolling/drawing conditions which can suppress grain growth of a nickel-free high-nitrogen stainless steel to manufacture a nickel-free high-nitrogen stainless steel material having a fine grain structure, and thus the present invention was accomplished based on these technical findings.

That is, a method of rolling/drawing a nickel-free high-nitrogen stainless steel material of the present invention is characterized in that the method includes: an intermediate annealing process in which a nickel-free high-nitrogen stainless steel material made of a fine grain structure having a maximum crystal grain size of 30 µm or less is annealed at a temperature of 900°C or higher and 1000°C or lower and then is air-cooled to room temperature; a rolling/drawing process in which the nickel-free high-nitrogen stainless steel material is extended while being thinned or being reduced in diameter after the intermediate annealing process; and a final solution treatment process in which the nickel-free high-nitrogen stainless steel material is heated to a temperature of 1200°C or higher and 1400°C or lower and then is air-cooled to room temperature.

In the method of rolling/drawing the nickel-free high-nitrogen stainless steel material, it is preferable that a rolling/drawing ratio represented by (wall thickness after working) / (wall thickness before working) be 10% or more and 50% or less. Further, it is preferable that the annealing be performed under an inert gas atmosphere in the intermediate annealing process. Furthermore, it is preferable that the intermediate annealing process and the rolling/drawing process be alternately repeated two or more times.

A method of manufacturing a thin seamless tube for a nickel-free high-nitrogen stainless steel stent of the present invention is characterized in that the method includes: performing plastic working on a nickel-free high-nitrogen stainless steel raw tube made of a fine grain structure having a maximum crystal grain size of 30 µm or less according to the above-described method of rolling/drawing the nickel-free high-nitrogen stainless steel material.

In the method of manufacturing the thin seamless tube for the nickel-free high-nitrogen stainless steel stent, it is preferable to perform a rolling/drawing in which one end of a raw tube of nickel-free high-nitrogen stainless steel is pushed into a hole of a die and pass to an opposite side of the hole, a plug is inserted into the raw tube in a state where the one end of the raw tube passes to the opposite side of the hole, and then the raw tube is drawn in a straight form by pulling out the one end.

A thin seamless tube for a nickel-free high-nitrogen stainless steel stent of the present invention is characterized by a tube having an outer diameter of 1.0 mm or larger and 6.0 mm or smaller, a wall thickness of 100 µm or thicker and 150 µm or thinner, and a length of 10 mm or longer and 2000 mm or shorter and in that the thin seamless tube is made of a fine grain structure having a maximum crystal grain size of 30 µm or less.

### Advantageous Effects of Invention

According to the method of rolling/drawing the nickel-free high-nitrogen stainless steel material of the present invention, the grain growth is suppressed, and thus it is possible to roll and draw the nickel-free high-nitrogen stainless steel material made of the fine grain structure having the maximum crystal grain size of 30 µm or less.

According to the method of manufacturing the thin seamless tube for the nickel-free high-nitrogen stainless steel stent of the present invention, the grain growth is suppressed, and thus it is possible to manufacture the thin seamless tube for the nickel-free high-nitrogen stainless steel stent made of the fine grain structure having the maximum crystal grain size of 30 µm or less.

According to the thin seamless tube for the nickel-free high-nitrogen stainless steel stent of the present invention, even in the complicated shape, it is possible to easily produce the thin seamless tube by the laser machining and to furthermore obtain the thin seamless tube almost free from defects.

### Brief Description of Drawings

Fig. 1 is a flowchart illustrating an example of a method of rolling/drawing a nickel-free high-nitrogen stainless steel material of the present invention.
Figs. 2(a) to 2(c) are diagrams illustrating examples of a thin seamless tube for a nickel-free high-nitrogen stainless steel stent of the present invention.
Fig. 3 is a flowchart illustrating an optimization testing process of an annealing temperature.
Fig. 4 is a cross-sectional microstructure of a cold-rolled high-nitrogen stainless steel sheet.
Fig. 5 is a measurement result of Vickers hardness of a cold-rolled high-nitrogen stainless steel sheet.
Fig. 6 is a flowchart illustrating a testing process of investigating an influence of repetition annealing.
Fig. 7 is a cross-sectional microstructure of a cold-rolled high-nitrogen stainless steel sheet.
Fig. 8 is a cross-sectional microstructure of a cold-rolled high-nitrogen stainless steel sheet.
Fig. 9 is a measurement result of Vickers hardness of a cold-rolled high-nitrogen stainless steel sheet.
Fig. 10 is a measurement result of Vickers hardness of a cold-rolled high-nitrogen stainless steel sheet.
Fig. 11 is a cross-sectional microstructure (annealing temperature - time - atmosphere) of a thin tube for 23Cr steel stent.
Fig. 12 is a cross-sectional microstructure (annealing temperature - time - atmosphere) of a thin tube for 23Cr steel stent.
Fig. 13 is an electron microscope image illustrating a cross-section (vertical direction) of a thin seamless tube.
Fig. 14 is an optical microscope image illustrating a cross-section (horizontal direction) of a thin seamless tube.

### Description of Embodiments

Hereinafter, a method of rolling/drawing a nickel-free high-nitrogen stainless steel material of the present invention, a thin seamless tube for a nickel-free high-nitrogen stainless steel stent, and a method of manufacturing the same will be described based on an embodiment with reference to the accompanying drawings.

### <Method of rolling/drawing nickel-free high-nitrogen stainless steel material>

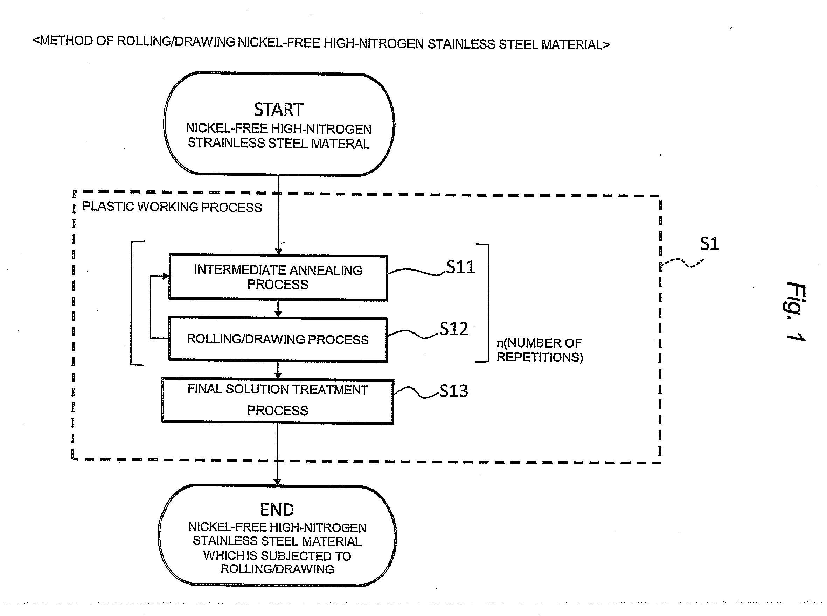

Fig. 1 is a flowchart illustrating an example of a method of rolling/drawing a nickel-free high-nitrogen stainless steel material of the present invention.

As illustrated in Fig. 1, the method of rolling/drawing the nickel-free high-nitrogen stainless steel material includes a plastic working process S1 in which the nickel-free high-nitrogen stainless steel material is subjected to plastic working. The plastic working process S1 includes an intermediate annealing process S11, a rolling/drawing process S12 after the intermediate annealing process S11, and a final solution treatment process S13. A chemical composition of the nickel-free high-nitrogen stainless steel material contains, for example, 20 to 25 mass% of Cr, 0.5 to 2 mass% of N, and a balance consisting of Fe and inevitably impurities. Examples of the inevitably impurities include Mo, Mn, C, and Si.

### (Intermediate annealing process S11)

Examples of the nickel-free high-nitrogen stainless steel material include a square form or a columnar form having a fine grain structure in which the maximum crystal grain size is 30 µm or less. The intermediate annealing process S11 is a process in which the nickel-free high-nitrogen stainless steel material is annealed at a temperature of 900°C or higher and 1000°C or lower and then is air-cooled to room temperature. The annealing may be performed in an oven or the like. When the annealing is performed at a temperature of 900°C or higher and 1000°C or lower, Vickers hardness can be reduced to suppress an increase of a grain size of the fine grain structure. When the annealing temperature is lower than 900°C, it is difficult to reduce the Vickers hardness and to suppress the increase of a grain size of the fine grain structure. In addition, when the annealing temperature is higher than 1000°C and is lower than 1060°C, the Vickers hardness is high, and thus the working becomes difficult. Moreover, when the annealing temperature is 1060°C or higher and lower than 1150°C, the Vickers hardness is high and nitrogen escapes, and thus it is difficult to become a single phase. Furthermore, when the annealing temperature is 1150°C or higher, nitrogen escapes, and thus it is difficult to become almost the single phase.

An annealing time is preferably 1 minute or longer and 30 minutes or shorter and more preferably, is 3 minutes or longer and 10 minutes or shorter. When the annealing time is shorter than 1 minute, an annealing effect is insufficient and becomes non-uniform. In addition, the annealing is preferably performed in an inert gas atmosphere and more preferably, is performed in a nitrogen atmosphere. When the annealing is performed in these atmospheres, it is possible to suppress denitrification from the nickel-free high-nitrogen stainless steel material. When the annealing is performed in a hydrogen atmosphere, hydrogen reacts with nitrogen in the nickel-free high-nitrogen stainless steel material, and thus the nitrogen content in the material significantly decreases.

### (Rolling/drawing process S12)

The rolling/drawing process S12 is a process in which the material is extended while being thinned or being reduced in diameter. For example, the square material is worked to be a sheet material by rolling with a rolling machine. In addition, one end of a raw tube of the nickel-free high-nitrogen stainless steel was pushed into a hole of a die and passed to an opposite side of the hole, a plug for holding an inner diameter was inserted into the raw tube in the state where one end of the raw tube passed to the opposite side of the hole, and then the raw tube was drawn in a straight form by pulling out one end. By doing this, the raw tube can be extended while being reduced in diameter. Generally, the rolling/drawing can be performed at room temperature.

In the rolling/drawing process S12, a rolling/drawing ratio represented by (wall thickness after working) / (wall thickness before working) is preferably 10% or more and 50% or less. When the rolling/drawing ratio exceeds 50%, there is a possibility that cracks occur in the material. When the rolling/drawing ratio is less than 10%, manufacturing efficiency is reduced.

Preferably, the intermediate annealing process S11 and the rolling/drawing process S12 are performed plural times as one set. The material can be extended while being thinned or being reduced in diameter little by little through a plurality of operations, and thus a value of a diameter, length, wall thickness or the like can be precisely controlled.

### (Final solution treatment process S13)

The final solution treatment process S13 is a process in which the material is annealed at a temperature of 1200°C or higher and 1400°C or lower and then is air-cooled to room temperature. The Vickers hardness can be improved by the solution treatment.

In addition, a non-magnetic material of γ-single phase can be obtained by the solution treatment. Since a magnetic material causes artifacts at the time of MRI measurement, it is undesirable to use as a biomaterial.

### <Seamless thin tube for nickel-free high-nitrogen stainless steel stent >

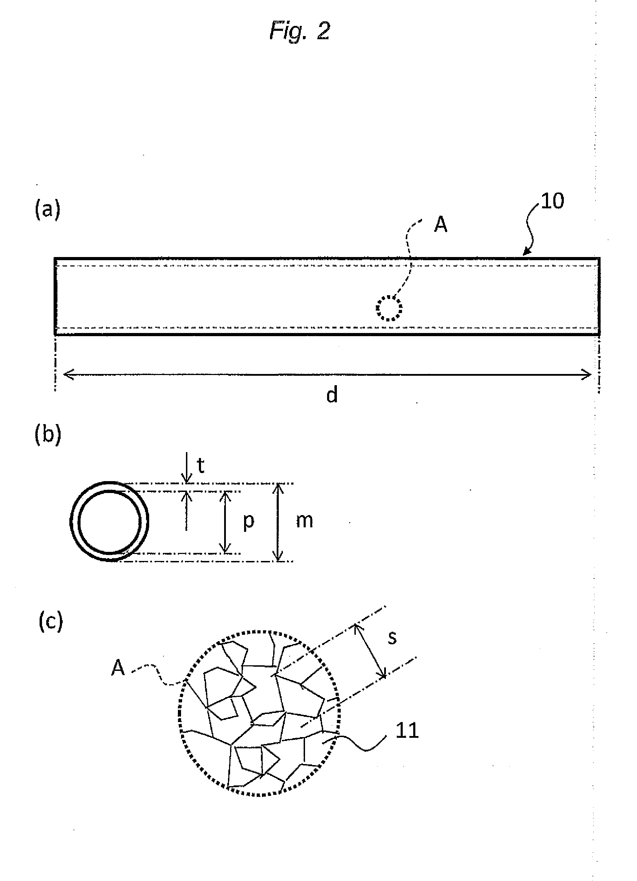

Figs. 2(a) to 2(c) are diagrams illustrating examples of a thin seamless tube for a nickel-free high-nitrogen stainless steel stent of the present invention. Fig. 2(a) is a plan view, Fig. 2(b) is a left-side view, and Fig. 2(c) is an enlarged schematic view of an "A" part of Fig. 2(a).

A thin seamless tube 10 for a nickel-free high-nitrogen stainless steel stent is defined to have a length "d" of 10 mm or longer and 2000 mm or shorter, an outer diameter "m" of 1.0 mm or larger and 6.0 mm or smaller, and a wall thickness "t" of 100 µm or thicker and 150 µm or thinner. From the defined conditions, an inner diameter "p" is also automatically defined. By making these sizes, it is possible to form a stent, which is capable for being inserted into a blood vessel having a diameter of 1 to 6 mm and holds constant durability, by laser machining.

The thin seamless tube 10 for the nickel-free high-nitrogen stainless steel stent is formed of a high-nitrogen stainless steel. Therefore, this is realized as a high-strength material with excellent corrosion properties in a living-body simulation environment. In addition, the thin seamless tube 10 for the nickel-free high-nitrogen stainless steel stent is also nickel-free; For this reason, this is realized as an anti-Ni allergic material.

As illustrated in Fig. 2(c), the thin seamless tube 10 for the nickel-free high-nitrogen stainless steel stent is formed from a fine grain structure 11. Since the thin seamless tube is formed from the fine grain structure 11, processing accuracy can be improved and thus it is easily produced even for a stent having a complicated shape. A maximum size "s" of the crystal grain of the fine grain structure 11 is 30 µm or less. The maximum size "s" is preferably 15 µm or less, and more preferably is 10 µm or less. As the maximum size "s" becomes smaller, cracks generated by the laser machining can be reduced, and thus mechanical properties of the stent after being processed are improved.

### <Method of manufacturing thin seamless tube for nickel-free high-nitrogen stainless steel stent>

A method of manufacturing a thin seamless tube for the nickel-free high-nitrogen stainless steel stent is a method in which a nickel-free high-nitrogen stainless steel raw tube is subjected to the plastic working according to the method of rolling/drawing the nickel-free high-nitrogen stainless steel material as described above.

First, for example, a squared-shaped test material is subjected to a raw tube forming (machining) process including a columnar shape machining process, a punching process using a gun drill, and a honing process, and thus the nickel-free high-nitrogen stainless steel raw tube is produced. Further, the honing process is a grinding method used in precision finishing and, for example, is a process of polishing an inner surface of a hole using a tool called a hone which is formed by attaching several rectangular grinding stones to a side face of a columnar rotating tool.

### (Intermediate annealing process)

The intermediate annealing process is a process in which the obtained nickel-free high-nitrogen stainless steel raw tube is annealed at a temperature of 900°C or higher and 1000°C or lower and then is air-cooled to room temperature. The annealing may be performed in an oven or the like. When the annealing is performed at a temperature of 900°C or higher and 1000°C or lower, Vickers hardness can be reduced to suppress an increase of a grain size of the fine grain structure. The annealing time is preferably 1 minute or longer and 30 minutes or shorter and more preferably, is 3 minutes or longer and 10 minutes or shorter.

### (Rolling/drawing process)

After the intermediate annealing process, the raw tube is extended while being reduced in diameter in the rolling/drawing process. For example, one end of a raw tube of the nickel-free high-nitrogen stainless steel was pushed into a hole of a die and passed to the opposite side of the hole, a plug for holding an inner diameter was inserted into the raw tube in the state where one end of the raw tube passed to the opposite side of the hole, and then the raw tube was drawn in a straight form by pulling out one end. By doing this, the raw tube can be extended while being reduced in diameter. Generally, the rolling/drawing can be performed at room temperature.

In the rolling/drawing process, a rolling/drawing ratio represented by (wall thickness after working) / (wall thickness before working) is preferably 10% or more and 50% or less. When the rolling/drawing ratio exceeds 50%, there is a possibility that cracks occur in the raw tube. When the rolling/drawing ratio is less than 10%, manufacturing efficiency is reduced.

Preferably, the intermediate annealing process and the rolling/drawing process are performed plural times as one set. The raw tube is extended while being reduced in diameter little by little through a plurality of operations, and thus a value of a diameter, length, wall thickness or the like can be precisely controlled.

### (Final solution treatment process)

The final solution treatment process is a process in which the material is annealed at a temperature of 1200°C or higher and 1400°C or lower and then the annealed material is air-cooled to room temperature. The Vickers hardness can be improved while maintaining the fine crystal grains by the solution treatment. In addition, a non-magnetic material of γ (austenite)-single phase can be obtained.

The method of rolling/drawing the nickel-free high-nitrogen stainless steel material as described above includes the intermediate annealing process S11 in which the nickel-free high-nitrogen stainless steel material made of the fine grain structure having the maximum crystal grain size of 30 µm or less is annealed at a temperature of 900°C or higher and 1000°C or lower and then is air-cooled to room temperature, the rolling/drawing process S12 in which the nickel-free high-nitrogen stainless steel material is extended while being thinned or being reduced in diameter after the intermediate annealing process S11, and the final solution treatment process S13 in which the material is heated to a temperature of 1200°C or higher and 1400°C or lower and then is air-cooled to room temperature. Therefore, it is possible to roll and draw the nickel-free high-nitrogen stainless steel material made of the fine grain structure 11 having the maximum crystal grain size of 30 µm or less while suppressing the grain growth.

In the method of rolling/drawing the nickel-free high-nitrogen stainless steel material, it is possible to set the rolling ratio represented by (wall thickness after working)/(wall thickness before working) to be 10% or more and 50% or less in the rolling/drawing process S12. Therefore, the grain growth is more suppressed and the fine grain structure 11 having the maximum crystal grain size of 30 µm or less is formed.

Further, in the method of rolling/drawing the nickel-free high-nitrogen stainless steel material, since the annealing is performed under an inert gas atmosphere in the intermediate annealing process S11, the grain growth is more suppressed and the fine grain structure 11 having the maximum crystal grain size of 30 µm or less is formed.

Moreover, in the method of rolling/drawing the nickel-free high-nitrogen stainless steel material, since the intermediate annealing process S11 and the rolling/drawing process S12 can be alternately repeated two or more times, the grain growth is more suppressed and the fine grain structure 11 having the maximum crystal grain size of 30 µm or less is formed.

In the method of manufacturing the thin seamless tube 10 for the nickel-free high-nitrogen stainless steel stent, the nickel-free high-nitrogen stainless steel raw tube made of the fine grain structure having the maximum crystal grain size of 30 µm or less is subjected to the plastic working according to the method of rolling/drawing the nickel-free high-nitrogen stainless steel material as described above. Therefore, the grain growth is suppressed and the thin seamless tube for the nickel-free high-nitrogen stainless steel stent made of the fine grain structure 11 having the maximum crystal grain size of 30 µm or less is manufactured.

In the method of manufacturing the thin seamless tube 10 for the nickel-free high-nitrogen stainless steel stent, rolling/drawing in which one end of the raw tube of the nickel-free high-nitrogen stainless steel was pushed into the hole of a die and passed to the opposite side of the hole, the plug was inserted into the raw tube in the state where one end of the raw tube passed to the opposite side of the hole, and then the raw tube was drawn in a straight form by pulling out one end is performed. Therefore, the grain growth is suppressed and the fine grain structure 11 having the maximum crystal grain size of 30 µm or less is formed.

The thin seamless tube 10 for the nickel-free high-nitrogen stainless steel stent is a tube having the outer diameter of 1.0 mm or larger and 6.0 mm or smaller, the wall thickness of 100 µm or thicker and 150 µm or thinner, and the length of 10 mm or longer and 2000 mm or shorter and is made of the fine grain structure 11 having the maximum crystal grain size of 30 µm or less. Therefore, even in the complicated shape, it is possible to easily produce the thin seamless tube by the laser machining and to furthermore obtain the thin seamless tube almost free from defects.

The method of rolling/drawing the nickel-free high-nitrogen stainless steel material, the thin seamless tube for the nickel-free high-nitrogen stainless steel stent, and the method of manufacturing the same according to the present invention are not intended to be limited to the above embodiment. Various aspects can be employed for details. Although Examples are described, the method of rolling/drawing the nickel-free high-nitrogen stainless steel material, the thin seamless tube for the nickel-free high-nitrogen stainless steel stent, and the method of manufacturing the same according to the present invention are not limited to Examples.

### [Examples]

### (Test Example 1)

### <Optimization of annealing temperature>

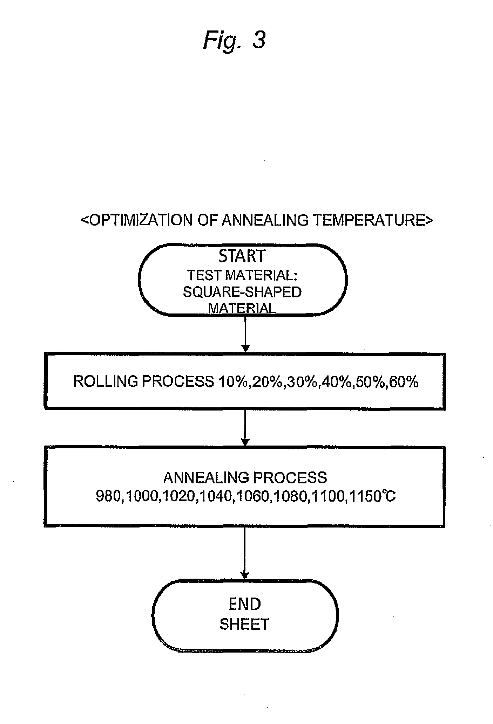

Fig. 3 is a flowchart illustrating an optimization testing process of an annealing temperature. First, plural nickel-free high-nitrogen stainless steel sheets having a sheet thickness of 5 mm were prepared as a test material. A chemical composition of the nickel-free high-nitrogen stainless steel was Fe - 23Cr - 1 Mo - 1N (in mass%). Each of the test materials was rolled at a rolling ratio of 10%, 20%, 30%, 40%, 50%, and 60%. The sheet thickness after rolling is indicated in Table 1.

**Table 1**

| Rolling ratio (%) | Sheet thickness after rolling (mm) | Note |
|---|---|---|
| 10 | 4.5 | - |
| 20 | 4 | - |
| 30 | 3.5 | - |
| 40 | 3 | - |
| 50 | 2.5 | - |
| 60 | - | Cracks occur in rolling. |

Next, except for a test material in which cracks occur at a rolling ratio of 60%, each of the test materials after rolling was subjected to a heat treatment at annealing temperatures of 980°C, 1000°C, 1020°C, 1040°C, 1060°C, 1080°C, 1100°C, and 1150°C, thereby preparing forty sample sheets for Test Example were prepared in total. Table 2 indicates Test Example No., sheet No., rolling ratios, and annealing temperatures.

**Table 2**

| Test Example No. | Sheet No. | Rolling ratio(%) | Annealing temperature(°C) |
|---|---|---|---|
| Test Example 1-1-1 | 1 | 10 | 980 |
| Test Example 1-1-2 | 2 | | 1000 |
| Test Example 1-1-3 | 3 | | 1020 |
| Test Example 1-1-4 | 4 | | 1040 |
| Test Example 1-1-5 | 5 | | 1060 |
| Test Example 1-1-6 | 6 | | 1080 |
| Test Example 1-1-7 | 7 | | 1100 |
| Test Example 1-1-8 | 8 | | 1150 |
| Test Example 1-2-1 | 9 | 20 | 980 |
| Test Example 1-2-2 | 10 | | 1000 |
| Test Example 1-2-3 | 11 | | 1020 |
| Test Example 1-2-4 | 12 | | 1040 |
| Test Example 1-2-5 | 13 | | 1060 |
| Test Example 1-2-6 | 14 | | 1080 |
| Test Example 1-2-7 | 15 | | 1100 |
| Test Example 1-2-8 | 16 | | 1150 |
| Test Example 1-3-1 | 17 | 30 | 980 |
| Test Example 1-3-2 | 18 | | 1000 |
| Test Example 1-3-3 | 19 | | 1020 |
| Test Example 1-3-4 | 20 | | 1040 |
| Test Example 1-3-5 | 21 | | 1060 |
| Test Example 1-3-6 | 22 | | 1080 |
| Test Example 1-3-7 | 23 | | 1100 |
| Test Example 1-3-8 | 24 | | 1150 |
| Test Example 1-4-1 | 25 | 40 | 980 |
| Test Example 1-4-2 | 26 | | 1000 |
| Test Example 1-4-3 | 27 | | 1020 |
| Test Example 1-4-4 | 28 | | 1040 |
| Test Example 1-4-5 | 29 | | 1060 |
| Test Example 1-4-6 | 30 | | 1080 |
| Test Example 1-4-7 | 31 | | 1100 |
| Test Example 1-4-8 | 32 | | 1150 |
| Test Example 1-5-1 | 33 | 50 | 980 |
| Test Example 1-5-2 | 34 | | 1000 |
| Test Example 1-5-3 | 35 | | 1020 |
| Test Example 1-5-4 | 36 | | 1040 |
| Test Example 1-5-5 | 37 | | 1060 |
| Test Example 1-5-6 | 38 | | 1080 |
| Test Example 1-5-7 | 39 | | 1100 |
| Test Example 1-5-8 | 40 | | 1150 |

Next, forty sample sheets for Test Example were subjected to microstructure observation and Vickers hardness measurement.

### <Results>

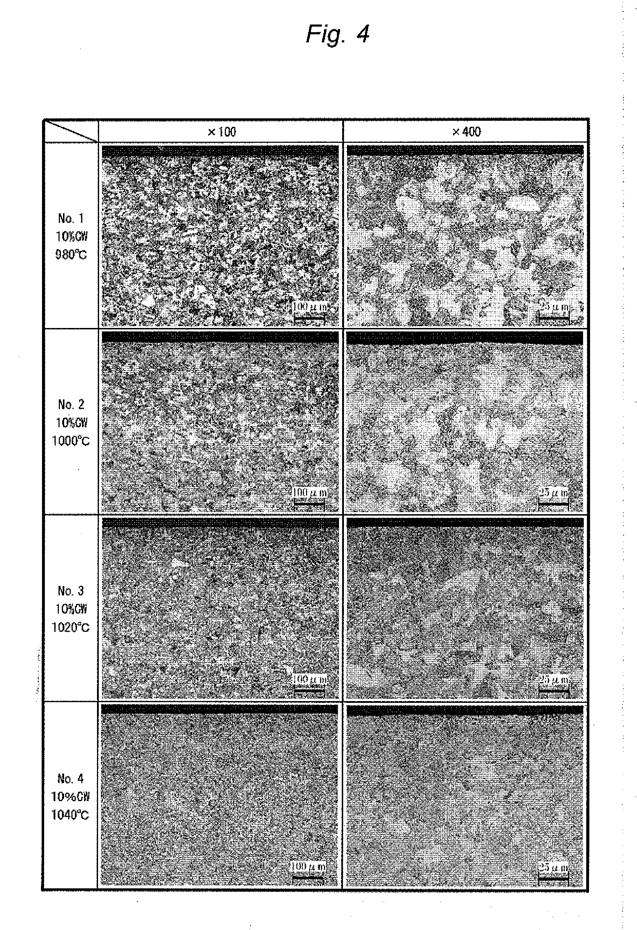

Fig. 4 illustrates a cross-sectional microstructure of a sample sheet for Test Example 1-1-1. Forty sample sheets for Test Example were compared with one another by the microstructure observation, but the difference due to the rolling ratio was not substantially seen.

However, the difference due to the difference in the annealing temperatures was significant, and the structure was significantly different between 980°C and 1040°C (low-temperature range) and between 1060°C to 1150°C (high-temperature region). When the heat treatment was performed at the low-temperature region, a pearlite-like structure of (α + Cr₂N) became large, and when the heat treatment was performed at the high-temperature, the pearlite-like structure disappeared and a single phase of γ (austenite) having a large crystal grain was formed. In addition, many Cr nitrides were precipitated at grain boundaries.

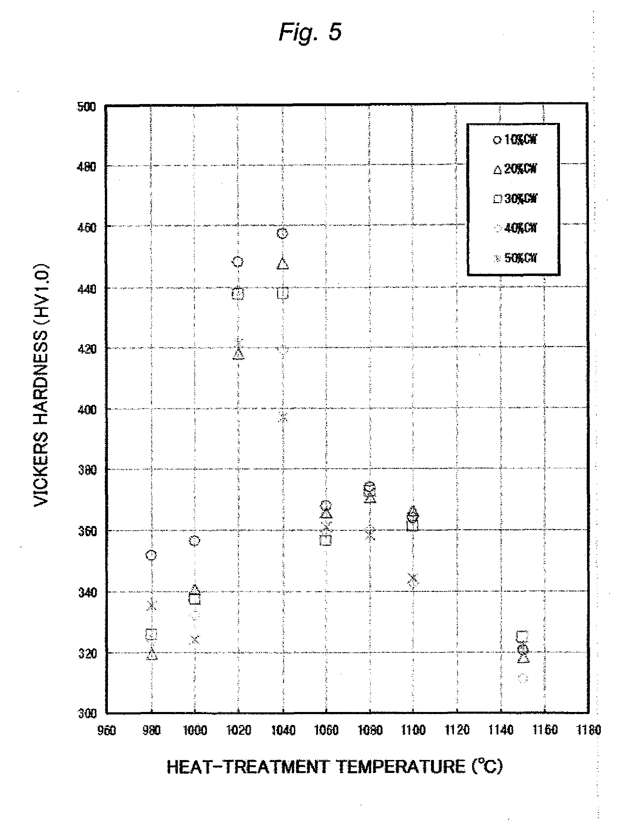

Fig. 5 illustrates measurement results of the Vickers hardness of forty sample sheets for Test Example. Peaks of the Vickers hardness were seen at 1020°C and 1040°C. Further, it was confirmed that variation in the Vickers hardness occurred at the low-temperature region but was small at the high-temperature region.

From the above results, it was found that the temperature range of 980°C or higher and 1000°C or lower and 1100°C or higher and 1150°C or lower, at which the Vickers hardness was reduced, was suitable for the annealing temperature. Two temperatures of 980°C and 1150°C were adopted as a candidate of the annealing temperature.

### (Test Example 2)

### <Effect investigation of repetition annealing>

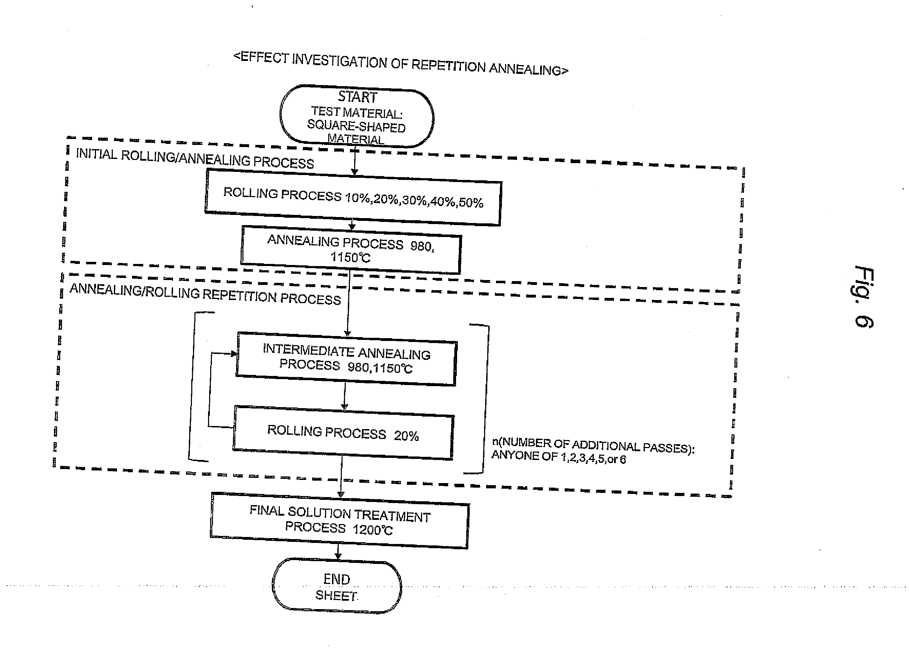

As in Test Example 1, plural high-nitrogen stainless steel sheets having a sheet thickness of 5 mm were prepared as a test material. Fig. 6 is a flowchart illustrating an effect investigation testing process of repetition annealing. The effect investigation test of the repetition annealing performed an initial rolling annealing process, an annealing/rolling repetition process, and a final solution treatment process as a series of processes. The initial rolling annealing process is the same as that of the optimization test of annealing temperature illustrated in Fig. 3. Meanwhile, since two temperatures of 980°C and 1150°C were employed as the candidates of annealing temperature, only samples annealed at those temperatures were subjected to a subsequent annealing/rolling repetition process. The annealing/rolling repetition process was a process in which treatment processes including an intermediate annealing process and a rolling process were repeated integer times from zero to six times. In addition, the intermediate annealing process was performed at either of 980°C or 1150°C, and the rolling ratio was only 20%. The final solution treatment process was an annealing process at 1200°C. The effect of the repetition annealing was investigated by the evaluation of the sample prepared through the series of processes described above.

Specifically, first, a sample which was subjected to the initial rolling annealing process under a condition of (rolling + 980°C x 10 min/air cooling) and a sample which was subjected to the initial rolling annealing process under a condition of (rolling + 1150°C x 10 min/air cooling) were subjected to the repetition rolling (hereinafter, referred to as an additional pass) under a condition of (980°C x 10 min/air cooling + cold rolling) and (1150°C x 10 min/air cooling + cold rolling) at the rolling ratio of 20%, respectively. The number of additional passes was integer times from zero to six times.

The number of re-rolling processes is 25 times (6 + 5 + 5 + 5 + 4 = 25) in total, and the number of samples in which the microstructure is observed after annealing is 60 samples. Further, when cracks occurred in the middle of the process, the sample was directly annealed and was subjected to the microstructure observation without subsequent rolling and annealing processes. The samples after each of the additional passes were given Test Example No. Table 3 illustrates Test Example No., sheet No., the number of additional passes, and the sheet thickness after the rolling.

**Table 3**

| Test Example No. | Sheet No. | Number of additional passes | Sheet thickness after rolling (mm) |
|---|---|---|---|
| Test Example 1-1-1 | 1 | zero | 4.5 |
| Test Example 1-1-8 | 8 | | |
| Test Example 1-1-1-1 | 1 | once | 3.6 |
| Test Example 1-1-8-1 | 8 | | |
| Test Example 1-1-1-2 | 1 | 2 times | 2.9 |
| Test Example 1-1-8-2 | 8 | | |
| Test Example 1-1-1-3 | 1 | 3 times | 2.3 |
| Test Example 1-1-8-3 | 8 | | |
| Test Example 1-1-1-4 | 1 | 4 times | 1.8 |
| Test Example 1-1-8-4 | 8 | | |
| Test Example 1-1-1-5 | 1 | 5 times | 1.5 |
| Test Example 1-1-8-5 | 8 | | |
| Test Example 1-1-1-6 | 1 | 6 times | 1.2 |
| Test Example 1-1-8-6 | 8 | | |
| Test Example 1-2-1 | 9 | zero | 4 |
| Test Example 1-2-8 | 16 | | |
| Test Example 1-2-1-1 | 9 | once | 3.2 |
| Test Example 1-2-8-1 | 16 | | |
| Test Example 1-2-1-2 | 9 | 2 times | 2.6 |
| Test Example 1-2-8-2 | 16 | | |
| Test Example 1-2-1-3 | 9 | 3 times | 2 |
| Test Example 1-2-8-3 | 16 | | |
| Test Example 1-2-1-4 | 9 | 4 times | 1.3 |
| Test Example 1-2-8-4 | 16 | | |
| Test Example 1-2-1-5 | 9 | 5 times | 1 |
| Test Example 1-2-8-5 | 16 | | |
| | | | |

| Test Example No. | Sheet No. | Number of additional passes | Sheet thickness after rolling (mm) |
|---|---|---|---|
| Test Example 1-3-1 | 17 | zero | 3.5 |
| Test Example 1-3-8 | 24 | | |
| Test Example 1-3-1-1 | 17 | once | 2.8 |
| Test Example 1-3-8-1 | 24 | | |
| Test Example 1-3-1-2 | 17 | 2 times | 2.2 |
| Test Example 1-3-8-2 | 24 | | |
| Test Example 1-3-1-3 | 17 | 3 times | 1.8 |
| Test Example 1-3-8-3 | 24 | | |
| Test Example 1-3-1-4 | 17 | 4 times | 1.4 |
| Test Example 1-3-8-4 | 24 | | |
| Test Example 1-3-1-5 | 17 | 5 times | 1.2 |
| Test Example 1-3-8-5 | 24 | | |
| | | | |

| Test Example No. | Sheet No. | Number of additional passes | Sheet thickness after rolling (mm) |
|---|---|---|---|
| Test Example 1-4-1 | 25 | zero | 3 |
| Test Example 1-4-8 | 32 | | |
| Test Example 1-4-1-1 | 25 | once | 2.4 |
| Test Example 1-4-8-1 | 32 | | |
| Test Example 1-4-1-2 | 25 | 2 times | 1.9 |
| Test Example 1-4-8-2 | 32 | | |
| Test Example 1-4-1-3 | 25 | 3 times | 1.5 |
| Test Example 1-4-8-3 | 32 | | |
| Test Example 1-4-1-4 | 25 | 4 times | 1.2 |
| Test Example 1-4-8-4 | 32 | | |
| Test Example 1-4-1-5 | 25 | 5 times | 1 |
| Test Example 1-4-8-5 | 32 | | |
| Test Example 1-5-1 | 33 | zero | 2.5 |
| Test Example 1-5-8 | 40 | | |
| Test Example 1-5-1-1 | 33 | once | 2 |
| Test Example 1-5-8-1 | 40 | | |
| Test Example 1-5-1-2 | 33 | 2 times | 1.6 |
| Test Example 1-5-8-2 | 40 | | |
| Test Example 1-5-1-3 | 33 | 3 times | 1.3 |
| Test Example 1-5-8-3 | 40 | | |
| Test Example 1-5-1-4 | 33 | 4 times | 1 |
| Test Example 1-5-8-4 | 40 | | |

| | | | |
|---|---|---|---|
| Annealing temperature of additional pass = 980°C and 1150°C | | | |

### <Final annealing>

After the repetition rolling, the samples were subjected to heat treatment/air cooling under a condition of (1200°C x 10 min) as a final solution treatment (also referred to as a final solution treatment). Non-magnetic samples of γ-single phase were obtained by the final solution treatment. As described above, the reason is that a magnetic biomaterial is undesirable.

With respect to the samples for Test Examples which were subjected to the above processes, the microstructure observation and the Vickers hardness measurement were performed.

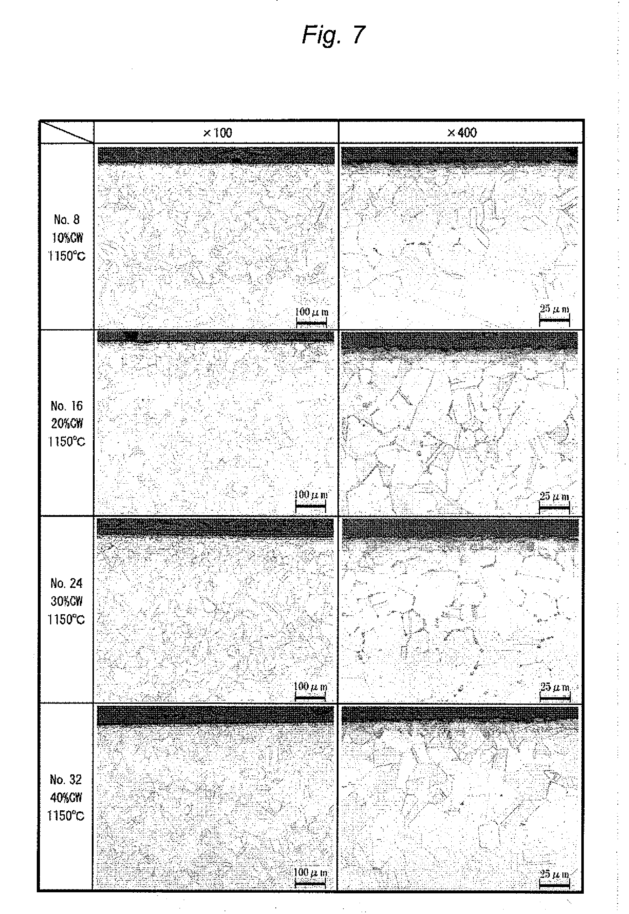

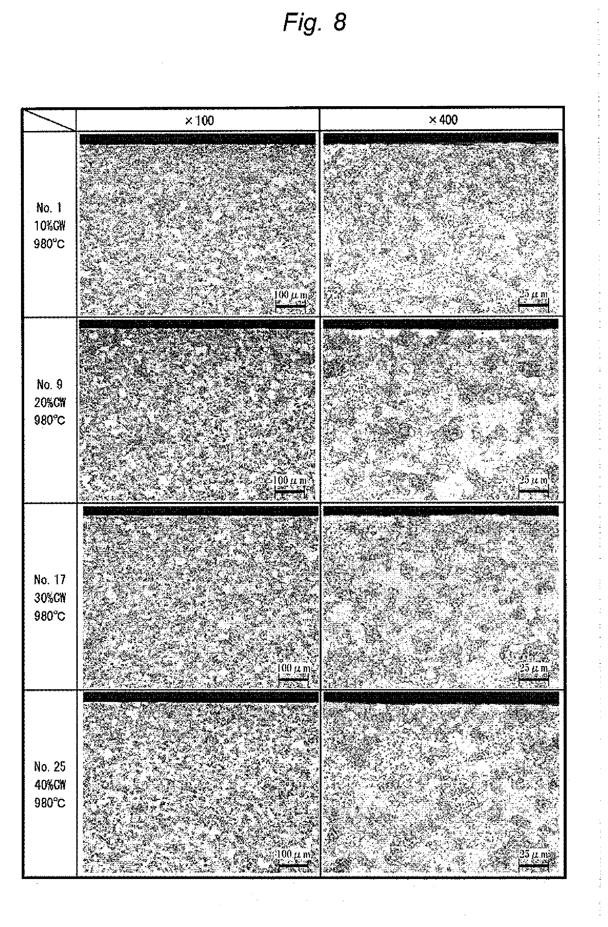

Fig. 7 and Fig. 8 illustrate examples of observation results of the microstructure of the samples after each of the additional passes. Regarding the repetition rolling, the microstructure was not significantly turned.

In addition, the microstructure of the samples which was subjected to the final solution treatment was observed. The pearlite-like structure of (α + Cr₂N) formed by the annealing at 980°C completely disappeared by the final solution treatment at 1200°C. Further, it was confirmed that the samples were non-magnetic. However, in some samples, it was observed that Cr nitrides were precipitated at grain boundaries.

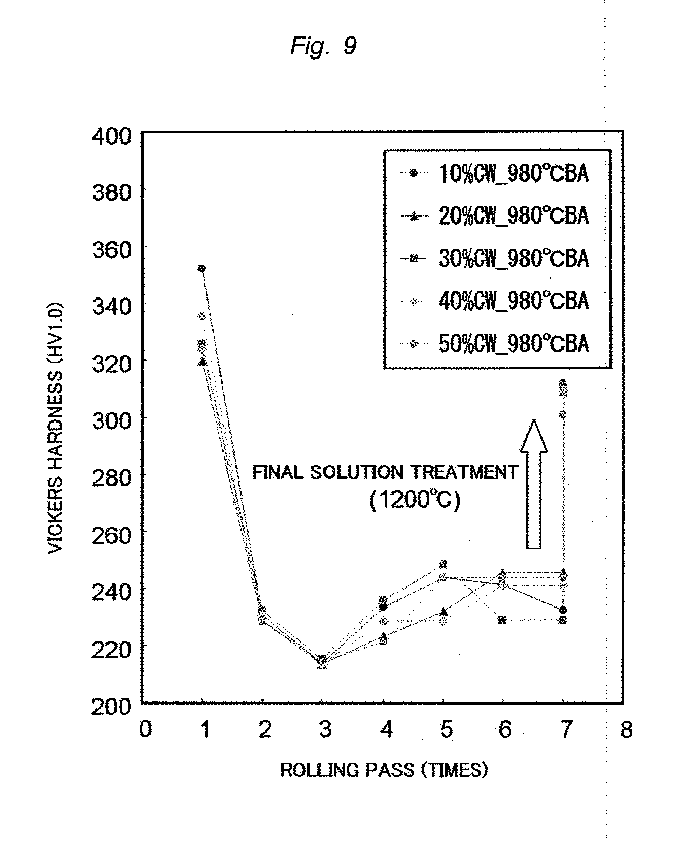

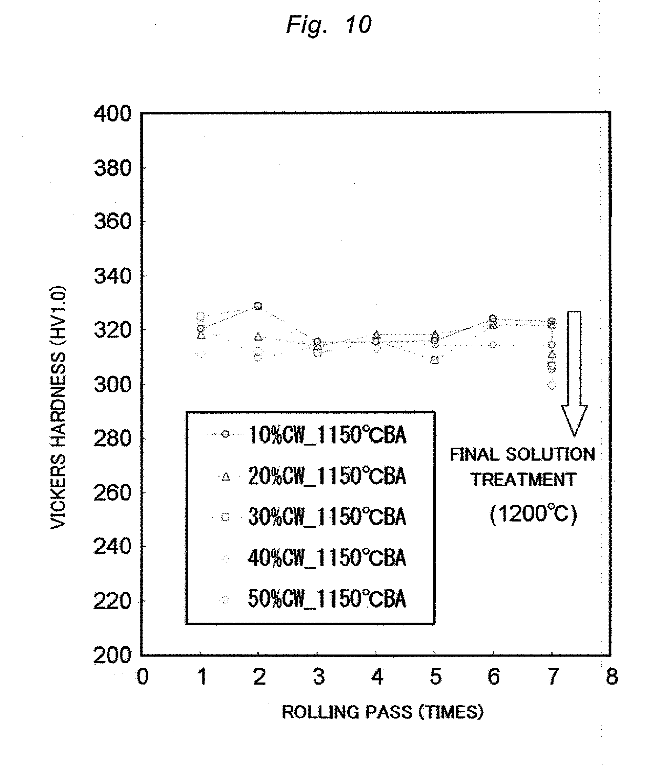

Measurement results of the Vickers hardness are illustrated in Fig. 9 and Fig. 10. As illustrated in Fig. 9, the Vickers hardness decreased significantly from 340 HV to 230 HV in a case where the intermediate annealing and the cold rolling (additional pass) were performed once at 980°C and the number of rolling processes was 2 times in total. Thereafter, even when the additional pass was repeated, the Vickers hardness was still in a decreased state. On the other hand, after the final solution treatment, the Vickers hardness rose significantly from 230 HV to 310 HV.

In contrast, as illustrated in Fig. 10, the Vickers hardness became constant at about 320 HV without being substantially changed even after the process of intermediate annealing + cold rolling (additional pass) was repeatedly performed at 1150°C. After the final solution treatment, the Vickers hardness was decreased from 320 HV to 300 HV.

### <Identification of crystal structure by X-ray diffraction>

Next, with respect to the pearlite-like structure (α + Cr₂N) which appeared at the time of repeatedly performing the intermediate annealing and the cold rolling (additional pass) at 980°C, identification and quantitative analysis were performed. An analyzing device used and analyzing conditions are indicated in Tables 4 and 5.

**Table 4**

| Analyzing device | Microfocused X-ray diffractometer (RINT-RAPID II made by Rigaku | |
|---|---|---|
| Analyzing conditions | Tube bulb | Co |
| | Monochromatization | Use monochrometer (Kα) |
| | Output of tube bulb | 40 kV - 30 mA |
| | Detector | Imaging plate (two dimension) |
| | (Reflection method) | |
| | Collimator | φ 300 µm |
| | ω angle | 22° - 25° (Reflective sample stand) |
| | φ angle | Oscillation ± 60° |
| | Measurement time | 30 min |
| | (exposure) | |

**Table 5**

| Analyzing device | X-ray diffractometer (RAD-RU 300 made by Rigaku Denki Co., Ltd.) | |
|---|---|---|
| Analyzing conditions | Target | Co |
| | Monochromatization | Use monochrometer (Kα ray) |
| | Output of target | 40 kV - 200 mA |
| | (Continuous measurement) | θ/2θ scanning |
| | Slit | Divergence 1°, Scattering 1°, Light- |
| | | receiving 0.3 mm |
| | Monochrometer light-receiving slit | 0.6 mm |
| | Scanning speed | 2° / min |
| | Sampling width | 0.02° |
| | Measurement angle | 30° - 130° |

### <Results>

Table 6 indicates identification results of the sample (No.9 - sixth additional pass), which is repeatedly subjected to the intermediate annealing and the cold rolling at 980°C, by X-ray diffraction. In addition, Table 7 indicates simple quantitative results.

**Table 6**

| Sample name | Identified substance | | ICDD card No. |
|---|---|---|---|
| No. 9 Sixth additional pass (980°C) | α-Fe | α-phase | 06-0696 |
| | C_{0.055}Fe_{1.945} | α'-phase : Martensite | 44-1290 |
| | Cr₂N | | 35-0803 |

**Table 7**

| Sample name | α-phase | α'-phase | Cr₂N |
|---|---|---|---|
| No. 9 Sixth additional pass (980°C) | 85.1 vol% | 8.9 vol% | 6.0 vol% |

The Vickers hardness decreased significantly from 340 HV to 230 HV in the case where the intermediate annealing and the cold rolling (additional pass) was performed once at 980°C and the number of rolling processes was 2 times in total. In the sample, a precipitation temperature range is shifted, but γ (austenite) phase is stable at a high-temperature side and two-phase structure of α + Cr₂N is stable at an intermediate temperature range. Since the nickel-free high-nitrogen stainless steel having the γ (austenite) -single phase at 1200°C or higher was annealed at a low-temperature side of 980°C, it was considered that pearlite transformation occurred in two-phase structure of α + Cr₂N. Since nitrogen was almost not solid-dissolved in a ferrite (α) phase, it was considered that all of the nitrogen was precipitated as chromium nitride Cr₂N.

Even after the intermediate annealing and the rolling (additional pass) were repeatedly performed at 980°C, the fine grain structure was held and the Vickers hardness was low, and thus it was confirmed that the work was easy compared with the sample which was subjected to the annealing and the cold rolling at 1150°C. In addition, the ferrite structure disappeared and the γ (austenite) single phase was formed by the final solution treatment process at 1200°C, and thus it was confirmed that the strength increased. Cr₂N was entirely resolved to be solid-dissolved in a matrix of the γ (austenite) phase.

By repeatedly performing the annealing and the cold rolling (additional pass) at 980°C, it is possible to suppress coarsening of a recrystallized grain by a lower annealing temperature compared with the sample which is annealed at 1150°C. When the working and heat treatment conditions described above are applied to thin tube machining, it is possible to improve workability of a thin tube and to realize grain refining of a crystal grain by only the annealing temperature.

### (Example 1)

### <Test method (production of thin seamless tube)>

Two test materials (bars in which one side had a rectangular cross-section of 14.5 mm and a length was 300 cm) made of nickel-free high-nitrogen stainless steel (Fe-23Cr-1Mo-1N) were prepared. These test materials were successively subjected to a) raw tube machining, b) plastic working, and c) final solution treatment.

### a) Raw tube machining

The test material was subjected to a machining process (punching by a gun drill and honing process), and thus a raw tube was produced.

### b) Plastic working

After being subjected to the intermediate annealing, the obtained raw tube was subjected to the plastic working by drawing, and thus a thin seamless tube was produced. The intermediate annealing was performed at the temperature of 980°C under an argon gas atmosphere. Further, the plastic working is a method of working a substance, which is plastically deformed by an applying force, into various shapes. Cold working which is performed at room temperature is a process capable of obtaining high dimensional accuracy, and hot working is used in large-sized products or crude processing. In the plastic working by drawing, one end of a raw tube was pushed into a hole of a die and passed to the opposite side of the hole, a plug was inserted into the raw tube in the state where one end of the raw tube passed to the opposite side of the hole, and then the raw tube was drawn in a straight form by pulling out one end.

### c) Final solution treatment

Thereafter, a final solution treatment was performed at the temperature of 1220°C under a nitrogen atmosphere. In the case of performing the annealing under the nitrogen atmosphere, it was possible to suppress denitrification from a surface of a thin-walled tube. By the final solution treatment, the pearlite-like structure of (α + Cr₂N) was turned into the γ (austenite) single phase.

A final shape of the thin seamless tube was intended to have an outer diameter of 1.4 mm, a wall thickness of 150 µm, and roundness of 0.01 mm to slip down an inclined plate having a straightness of 5°. In addition, it was intended to produce a thin seamless tube having an uneven thickness of ≤ 0.01 mm, a surface roughness Ra satisfying a relation of ≤ 0.5 µm, and a length of 1000 mm or longer. Moreover, it was intended to finally obtain a thin seamless tube which was non-magnetic or was reduced as much as possible in magnetic property.

The microstructure was observed on the thin seamless tube which was subjected to the final solution treatment. The γ (austenite) single phase structure was confirmed. In addition, EPMA (Electron Probe MicroAnalyser) analysis and nitrogen analysis (infrared absorption method) were performed. It was confirmed that N was 0.91 mass% and the denitrification did not occur. Thus, it was possible to produce a normal product having the outer diameter of 1.4 mm and the length of 1000 mm.

Next, the intermediate annealing was performed under conditions given in Table 8, and changes in the microstructure of the thin seamless tube to be obtained were examined.

**Table 8**

| Temperature (°C) | Atmosphere | | | |
|---|---|---|---|---|
| | Ar | Vacuum | Air | N₂ |
| 980 | 6 min | - | - | - |
| 1150 | 10 min | - | 10, 30 min | 10, 20, 30 min |
| 1200 | 6 min | 10, 20, 30 min | - | 10, 20, 30 min |
| 1220 | 3, 6 min | - | - | - |
| 1230 | - | 6 min | - | - |

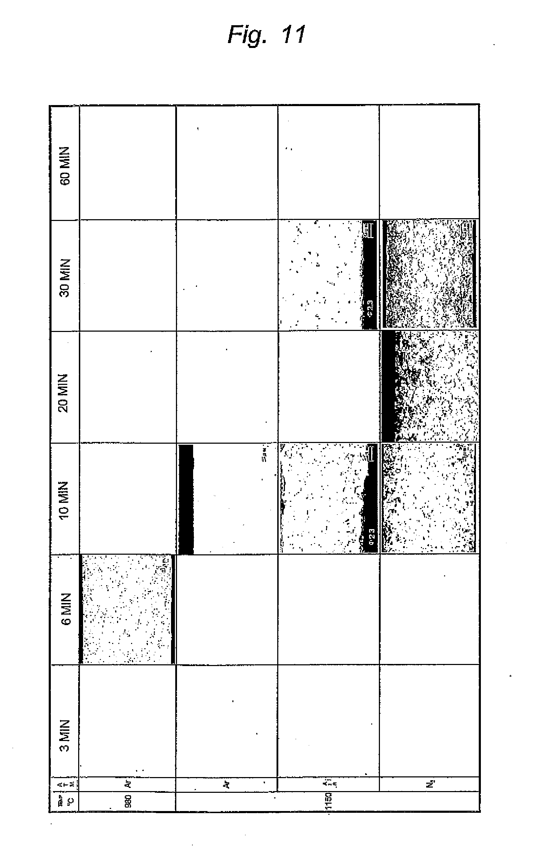

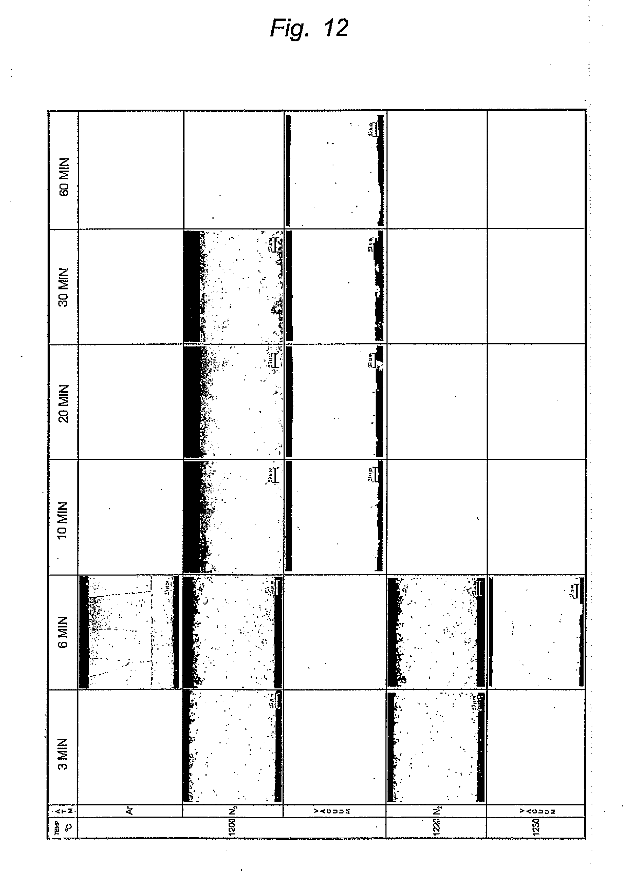

Figs. 11 and 12 illustrate results of the microstructure observation with respect to the sample (φ2.3 mm) before the final solution treatment and the sample after the final solution treatment. The condition of the final solution treatment was 1220°C x 6 minutes.

When the intermediate annealing was performed under an Ar atmosphere, the α (ferrite) single phase structure was formed. On the other hand, when the intermediate annealing was performed under a condition of 1200°C x 6 minutes, two-phase structure of α (ferrite) -γ (austenite) was formed.

When the intermediate annealing was performed under an air or a nitrogen atmosphere, an oxide film or a nitride film was formed on the surface, but an outward diffusion of nitrogen was suppressed, thereby forming γ (austenite) phase. In the intermediate annealing under the air atmosphere, surface corrosion of the sample intensely occurred. Accordingly, the condition of the final solution treatment was selected with respect to the sample which was subjected to the intermediate annealing under the nitrogen atmosphere. As the time of the intermediate annealing became longer, absorption of nitrogen from the surface of the sample increased. Consequently, the nitrogen was precipitated in the crystal grain boundaries and the crystal grains as a nitride, and thus the thickness of a nitride layer increased. In addition, as the intermediate annealing temperature became higher, the nitride of a center portion of a wall thickness was solid-dissolved. In order to solid-dissolve the nitride and to suppress the denitrification by a thin nitride layer on the surface, a condition of 1220°C x 6 minutes is selected as an optimum condition in the final solution treatment to form the structure of the γ (austenite) single phase.

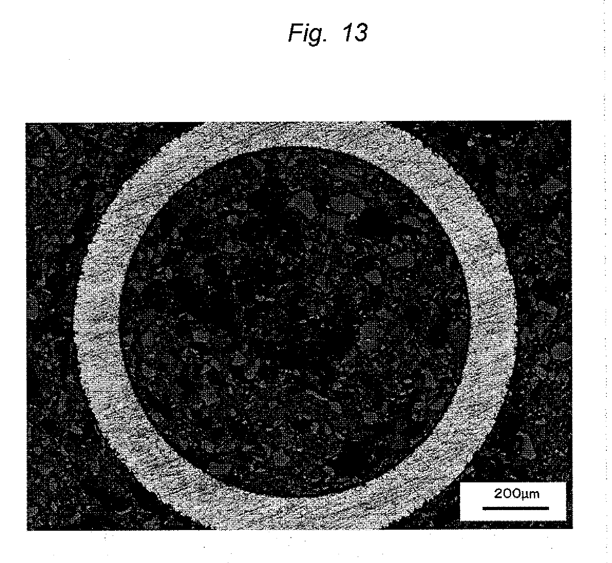

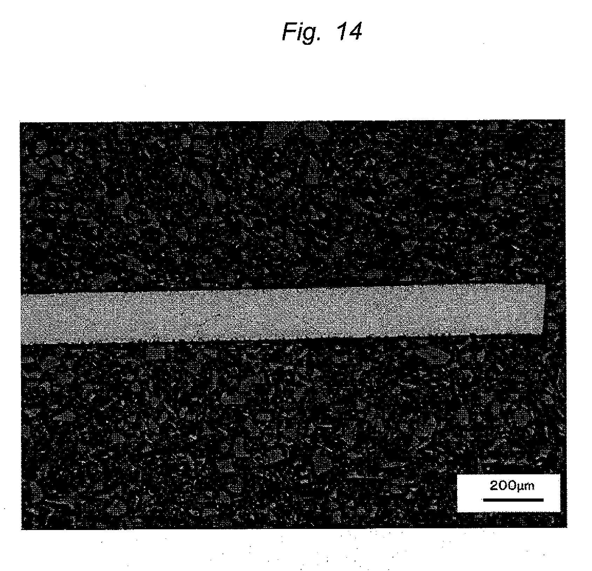

Fig. 13 is an electron microscope image illustrating a cross-section (vertical direction) of the seamless thin tube, and Fig. 14 is an optical microscope image illustrating a cross-section (horizontal direction) of the seamless thin tube.

It was founded that the target thin seamless tube was manufactured at the temperature of 1220°C for the treatment time of 3 to 10 minutes under the nitrogen atmosphere as optimum conditions in the final solution treatment.

### Industrial Applicability

The method of rolling/drawing the nickel-free high-nitrogen stainless steel material in which the increase of the grain size of the fine grain structure is suppressed, the thin seamless tube of the nickel-free high-nitrogen stainless steel made of the fine grain structure having the maximum crystal grain size of 30 µm or less, and the method of manufacturing the same are provided. The nickel-free high-nitrogen stainless steel material is expected to be used for not only the thin seamless tube for stent but also medical equipment such as orthodontic wires which have the complicated shape and require the high strength daily necessities or the like.

### Reference Signs List

S11: intermediate annealing process
S12: rolling/drawing process
S13: final solution treatment process
10: thin seamless tube for nickel-free high-nitrogen stainless steel stent
11: fine grain structure

## Claims

1. A method of rolling/drawing a nickel-free high-nitrogen stainless steel material, the method comprising:
an intermediate annealing process in which a nickel-free high-nitrogen stainless steel material made of a fine grain structure having a maximum crystal grain size of 30 µm or less is annealed at a temperature of 900°C or higher and 1000°C or lower and then is air-cooled to room temperature;
a rolling/drawing process in which the nickel-free high-nitrogen stainless steel material is extended while being thinned or being reduced in diameter after the intermediate annealing process; and
a final solution treatment process in which the nickel-free high-nitrogen stainless steel material is heated to a temperature of 1200°C or higher and 1400°C or lower and then is air-cooled to room temperature.

2. The method of rolling/drawing the nickel-free high-nitrogen stainless steel material according to claim 1, wherein a rolling/drawing ratio represented by (wall thickness after working)/(wall thickness before working) is 10% or more and 50% or less in the rolling/drawing process.

3. The method of rolling/drawing the nickel-free high-nitrogen stainless steel material according to claim 1 or 2, wherein the annealing is performed under an inert gas atmosphere in the intermediate annealing process.

4. The method of rolling/drawing the nickel-free high-nitrogen stainless steel material according to any one of claims 1 to 3, wherein the intermediate annealing process and the rolling/drawing process are alternately repeated two or more times.

5. A method of manufacturing a thin seamless tube for a nickel-free high-nitrogen stainless steel stent, the method comprising: performing plastic working on a nickel-free high-nitrogen stainless steel raw tube made of a fine grain structure having a maximum crystal grain size of 30 µm or less according to the method of rolling/drawing the nickel-free high-nitrogen stainless steel material according to any one of claims 1 to 4.

6. The method of manufacturing the thin seamless tube for the nickel-free high-nitrogen stainless steel stent according to claim 5, wherein drawing is performed by pushing one end of the nickel-free high-nitrogen stainless steel raw tube into a hole of a die and letting the one end into opposite side of the hole, inserting a plug into the raw tube in a state where the one end of the raw tube passes to the opposite side of the hole, and then drawing the raw tube in a straight form by pulling out the one end.

7. A thin seamless tube for a nickel-free high-nitrogen stainless steel stent having an outer diameter of 1.0 mm or larger and 6.0 mm or smaller, a wall thickness of 100 µm or thicker and 150 µm or thinner, and a length of 10 mm or longer and 2000 mm or shorter,
wherein the thin seamless tube is made of a fine grain structure having a maximum crystal grain size of 30 µm or less.
